# EUROPEAN PATENT SPECIFICATION

(11) **EP 2 139 520 B1**
(45) Date of publication and mention of the grant of the patent: **21.08.2013**
(21) Application number: 08795826.0
(22) Date of filing: 30.04.2008
(51) Int. Cl.: A61K 45/06, A61P 33/02

(54) **ALIPHATIC ACID-CONTAINING N-HALOGENATED AMINO ACID FORMULATIONS**
ALIPHATISCHE SÄUREHALTIGE N-HALOGENIERTE AMINOSÄURENFORMULIERUNGEN
FORMULATIONS D'ACIDES AMINÉS N-HALOGÉNÉS CONTENANT DE L'ACIDE ALIPHATIQUE

(30) Priority: 01.05.2007 US 915271 P
(43) Date of publication of application: 06.01.2010
(73) Proprietor: Alcon Research, Ltd., Fort Worth TX 76134 (US)
(72) Inventor: CHOWHAN, Masood A., Arlington, Texas 76016 (US); HAN, Wesley Wehsin, Arlington, Texas 76006 (US); SCHNEIDER, L. Wayne, Crowley, Texas 76036 (US); STROMAN, David W., Irving, Texas 75063 (US)
(74) Representative: Teipel, Stephan
(86) International application number: PCT/US2008/061950
(87) International publication number: WO 2008/134692

(56) References cited:
- WO-A-2007/044559
- GB-A- 2 072 371

## Description

### CROSS-REFERENCE TO RELATED APPLICATION

This application claims priority under 35 U.S.C. §119 to U.S. Provisional Patent Application No. 60/915,271 filed May 1, 2007, the entire contents of which are incorporated herein by reference.

### TECHNICAL FIELD OF THE INVENTION

The present invention relates to the use of an aliphatic acid for improving the antimicrobial properties of a formulation comprising N-halogenated amino acid compounds.

### BACKGROUND OF THE INVENTION

It is generally desirable to use the minimum quantity of an antimicrobial compound necessary to achieve desired effects. This is because undesirable side-effects are more probable when higher concentrations of an antimicrobial are used at a delivery site through the use of, for example, high concentration formulations, more frequent dosing, or longer-duration treatment. Unfortunately, while the use of lower concentrations of antimicrobial compounds generally helps to reduce the potential for undesirable effects, this practice increases the risk that the compounds may not achieve the required level of antimicrobial effect. Also, microbial resistance can develop quickly if antimicrobial compounds are not used at a sufficient concentration. Therefore, inventions that improve the antimicrobial activity of antimicrobial compounds are desirable as they allow for decreased concentrations of such compounds to be used at a delivery site, reducing the incidence and risk of undesired side effects and microbial resistance.

N-halogenated amino acid compounds are known to have desirable antimicrobial properties including antibacterial, anti-infective, antifungal, and/or antiviral properties. Many such N-halogenated amino acid compounds are disclosed in U.S. Patent Application Publication Nos. 2005/0065115 and 2006/0247209, the entire contents of which are incorporated by reference herein.

To cite one of many applications, the use of formulations having antimicrobial properties is important for the treatment of ophthalmic infections such as conjunctivitis. Conjunctivitis can be caused by various kinds of microbes, with most cases being due to bacteria and/or viruses. Unfortunately, conjunctivitis symptoms are not specific to the etiology of the infectious agent and significant testing may be required to determine the causative agent or microbe. Viral conjunctivitis, often caused by adenovirus, is highly contagious yet has no currently known efficacious treatment that provides other than symptom relief. Care must be taken in selecting appropriate agents for treating conjunctivitis, given the sensitive tissues affected by the infection. In view of the above-recited difficulties in treatment, formulations for treating conjunctivitis are needed that have broad-spectrum antimicrobial properties capable of treating bacteria, viruses, fungi, etc., a benign toxicological profile, and/or characteristics that prevent the transmission of contagious infectious agents.

Microbial resistance to conventional antimicrobial treatment is an ongoing concern to medical professionals. Until the problem of resistance is overcome, a steady supply of new treatments and therapies for treating microbial infections is required in order to blunt the effect of microbe mutations that render conventional therapies less effective or, in certain cases, ineffective.

### BRIEF SUMMARY OF THE INVENTION

The present invention relates to the use of an aliphatic acid for enhancing the antimicrobial activities of a formulation comprising N-halogenated amino acid compounds. The present inventors have discovered that the antimicrobial activity of N-halogenated amino acid compounds is enhanced in a formulation comprising an aliphatic acid compound such as sodium acetate.

The foregoing brief summary broadly describes the features and technical advantages of certain embodiments of the present invention. Additional features and technical advantages will be described in the detailed description of the invention that follows. Novel features which are believed to be characteristic of the invention will be better understood from the detailed description of the invention when considered in connection with any accompanying figures. However, figures provided herein are intended to help illustrate the invention or assist with developing an understanding of the invention.

### BRIEF DESCRIPTION OF THE DRAWINGS

A more complete understanding of the present invention and the advantages thereof may be acquired by referring to the following description, taken in conjunction with the accompanying drawings and wherein:
FIGURE 1 is a graph comparing the antimicrobial activity of 2,2-dimethyl-N,N-dichlorotaurine in various formulations against *S*. *aureus*; and
FIGURE 2 is a graph comparing the antimicrobial activity of 2,2-dimethyl-N,N-dichlorotaurine in various formulations against *C*. *albicans.*

### DETAILED DESCRIPTION OF THE INVENTION

### I. Definitions

Unless defined otherwise, technical and scientific terms used herein have the same meaning as commonly understood by one of ordinary skill in the art.

As used herein, the term "aliphatic acid" refers to any pharmaceutically acceptable compound comprising a carboxylic acid with a linear, branched, saturated or unsaturated hydrocarbon chain.

As used herein, the term "antimicrobial" refers to an ability to kill or inhibit the growth of microbes (to include, without limitation, bacterial, viruses, yeast, fungi, spores, protozoa, parasites, etc.), or to attenuate or eradicate a microbial infection.

As used herein, the term "subject" refers to either a human or to non-human domesticated or non-domesticated animals (such as primates, mammals, vertebrates, invertebrates, etc.). The terms "subject" and "patient" may be used interchangeably herein.

As used herein, the terms "treatment", "treating", and the like mean obtaining a desired pharmacologic and/or physiologic effect. The desired effect may be, without limitation, prevention of a disease or infection in certain usage and/or may be therapeutic in terms of a partial or complete cure for a disease or infection and/or adverse effect attributable to the disease or infection.

### II. Methods and Formulations

The N-halogenated amino acids have the following general formula: 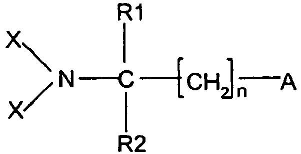 where X is one or more halogens and R1 and R2 are any of the nonpolar, uncharged polar, and charged polar amino acid and amino acid derivative side chains known to those of skill in the art. A represents an acid such as a carboxylic, sulfonic, phosphoric, boric or other acid known to those of skill in the art. There may be one or more carbon atoms between the amine and acid, and each carbon may contain on or more R substituents.

The preferred N-halogenated amino acids have the following structure: haloamino-stabilizer-linker-acid, where (a) the "haloamino" is either N-halogen or N,N-dihalogen (e.g., -NHCl or -NCl₂); (b) the "stabilizer" comprises sidechains attached to the carbon next to the haloamino group (e.g., hydrogen, -CH₃, lower alkyl, the group -COOH or a C₃₋₆ cycloalkyl ring); (3) the "linker" is either alkyl or cycloalkyl; and (d) the "acid" is one of the following: - COOH, -SO₃H, -P(=O)(OH)₂, -B(OH)₂ or hydrogen, and all the pharmaceutically acceptable salts of these acids generally known to those skilled in the art, including but not limited to sodium, potassium, calcium, etc.

The most preferred N-halogenated amino acids are 2,2-dimethyl-N,N-dichlorotaurine, analogs of 2,2-dimethyl-N,N-dichlorotaurine formed by replacement of the sulfonic acid group with carboxylic acid, phosphoric acid, borate, etc., 2,2-di alkyl-N,N-dichlorotaurine or 2,2-R-N,N-dichlorotaurine, where R is an aliphatic or aromatic side chain. Methyl groups of N-halogenated amino acids may be replaced with alkyl, aryl, benzyl, or other hydrocarbon cyclic or non-cyclic groups.

Generally, the aliphatic acid compound is a free acid or a metal salt, including but not limited to sodium, potassium, calcium, magnesium, etc. and all their states of hydration. Preferred aliphatic acids are acetate compounds such as sodium acetate, potassium acetate, calcium acetate, and magnesium acetate. Sodium acetate is particularly preferred. Also preferred is butyric acid. However, embodiments of the present invention may include other pharmaceutically acceptable aliphatic acids.

Certain methods and formulations comprise the use of N-halogenated amino acids with phase transfer agents to improve their antimicrobial properties. Co-pending U.S. Provisional Patent Application No. 60/915,291 entitled "N-HALOGENATED AMINO ACID FORMULATIONS," herein incorporated by reference in its entirety, discloses such N-halogenated amino acid formulations.

### III. Applications

The invention is particularly useful in treating mammalian and human subjects having or at risk of having a microbial tissue infection. Microbial tissue infections that may be treated or prevented using the present invention are referred to in J. P. Sanford et al., "The Sanford Guide to Antimicrobial Therapy 2007" 37th Edition (Antimicrobial Therapy, Inc.). Particular microbial tissue infections that may be treatable by using the present invention include those infections caused by bacteria, viruses, protozoa, fungi, yeast, spores, and parasites. The present invention is also particularly useful for antimicrobial formulations for and methods of treating ophthalmic, otic, dermal, upper respiratory, lung/lower respiratory, esophageal, and nasal/sinus infections.

Certain embodiments of the present invention are particularly useful for treating ophthalmic tissue infections. Examples of ophthalmic conditions that may be treated using the present invention include conjunctivitis, keratitis, blepharitis, dacyrocystitis, hordeolum and corneal ulcers. The invention may also be used prophylactically in various ophthalmic surgical procedures that create a risk of infection.

Otic and nasal/sinus tissue infections may also be treated using embodiments of the present invention. Examples of otic conditions that may be treated using the present invention include otitis externa and otitis media, including those situations where the tympanic membrane has ruptured or tympanostomy tubes have been implanted. Examples of nasal/sinus conditions that may be treated using the present invention include rhinitis, sinusitis, nasal carriage and situations where the nasal or sinus tissues are affected by surgery. Examples of respiratory infections and infectious agents include pneumonia, influenza, bronchitis, respiratory syncytial virus, etc.

Embodiments of the present invention may be used in disinfecting surfaces, particularly in healthcare-related structures such as hospitals, veterinary clinics, dental and medical offices, and for applications such as the sterilization of surgical instruments such as scalpels, electronic instrumentation, etc. Surgical instruments can be coated with certain formulations to provide for a sterile coating prior to surgery. Certain embodiments of the present invention may be used in the disinfection of public areas such as schools, public transportation facilities, restaurants, hotels and laundries and for the disinfection of household surfaces such as toilets, basins, and kitchen areas.

Certain formulations described herein may be used to disinfect and/or clean contact lenses in accordance with processes known to those skilled in the art and described in additional detail in co-pending U.S. Provisional Patent Application No. 60/970,634 entitled "N-HALOGENATED AMINO ACID FORMULATIONS AND METHODS FOR CLEANING AND DISINFECTION," herein incorporated by reference in its entirety. More specifically, contact lenses are removed from a patient's eyes and then immersed in such formulations for a time sufficient to disinfect the lenses. Disinfection and/or cleaning typically requires soaking the lenses in the formulation for approximately 4 to 6 hours.

Other embodiments of the present invention may also be useful in disinfection or treatment solutions for skin and body tissue surfaces of a subject, providing antimicrobial activity against bacteria, fungi, viruses, protozoa, etc. Such treatment may be prophylactic or may be used to treat infected body tissue or wounds having one or more varieties of infectious agents present. These embodiments may also be useful in treating the dermatological diseases caused by bacteria, fungi, viruses, protozoa, etc. Such embodiments may comprise formulations having one or more N-halogenated amino acids and aliphatic acids in a vehicle suitable for topical use. Disinfectant solutions for the skin are especially useful to disinfect hands, particularly in healthcare and unhygienic settings. Disinfection may also be useful in surgical settings, both for healthcare providers and to provide a clean field on a surgical subject.

Certain embodiments of the present invention may be useful in treating onychomycosis. Onychomycosis refers to the invasion of a nail plate by a fungus. The infection may be due to a dermatophyte, yeast, or nondermatophyte mold. The term "tinea unguium" is used specifically to describe invasive dermatophytic onychomycosis. Implicated dermatophytes include, but are not limited to: *Epidermophyton floccosum, Microsporum audouinii, Microsporum canis, Microsporum gypseum, Trichophyton mentagrophytes, Trichophyton rubrum, Trichophyton schoenleinii, Trichophyton tonsurans.* Additional fungi that may cause onychomycosis include, but are not limited to, *Acremonium* spp., *Aspergillus* spp., *Candida* spp., *Fusarium oxysporum, Scopulariopsis brevicaulis, Onychocola canadensis,* and *Scytalidium dimidiatum.*

Embodiments of the present invention may also be used prophylactically to prevent infection of a tissue by an infectious agent. In such embodiments, a tissue at risk of infection is contacted with a formulation of the present invention.

### IV. Pharmaceutics and Formulations

### A. Dosage

The phrase "pharmaceutically effective amount" is an art-recognized term, and refers to an amount of an agent that, when incorporated into a pharmaceutical formulation, produces some desired effect at a reasonable benefit/risk ratio applicable to any medical treatment. The effective amount may vary depending on such factors as the disease or infectious agent being treated, the particular formulation being administered, or the severity of the disease or infection agent.

The phrase "pharmaceutically acceptable" is art-recognized and refers to formulations, polymers and other materials and/or dosage forms which are suitable for use in contact with the tissues of human beings and animals without excessive toxicity, irritation, allergic response, or other problem or complication, commensurate with a reasonable benefit/risk ratio as determined by one of ordinary skill in the art.

In particular embodiments, a formulation is administered once a day. However, the formulations may also be formulated for administration at any frequency of administration, including once a week, once every 5 days, once every 3 days, once every 2 days, twice a day, three times a day, four times a day, five times a day, six times a day, eight times a day, every hour, or any greater frequency. Such dosing frequency is also maintained for a varying duration of time depending on the therapeutic regimen. The duration of a particular therapeutic regimen may vary from one-time dosing to a regimen that extends for months or years. One of ordinary skill in the art would be familiar with determining a therapeutic regimen for a specific indication. Factors involved in this determination include the disease to be treated, particular characteristics of the subject, and the particular antimicrobial formulation.

### B. Formulations

In addition to an N-halogenated amino acid and aliphatic acid, the formulations optionally comprise one or more excipients. Excipients commonly used in pharmaceutical formulations include, but are not limited to, tonicity agents, preservatives, chelating agents, buffering agents, surfactants and antioxidants. Other excipients comprise solubilizing agents, stabilizing agents, comfort-enhancing agents, polymers, emollients, pH-adjusting agents and/or lubricants. Any of a variety of excipients may be used including water, mixtures of water and water-miscible solvents, such as C1-C7-alkanols, vegetable oils or mineral oils comprising from 0.5 to 5% non-toxic water-soluble polymers, natural products, such as alginates, pectins, tragacanth, karaya gum, xanthan gum, carrageenin, agar and acacia, starch derivatives, such as starch acetate and hydroxypropyl starch, and also other synthetic products such as polyvinyl alcohol, polyvinylpyrrolidone, polyvinyl methyl ether, polyethylene oxide, preferably cross-linked polyacrylic acid and mixtures of these products. The concentration of the excipient is, typically, from 1 to 100,000 times the concentration of the N-halogenated amino acid and the aliphatic acid. In preferred embodiments, excipients are selected on the basis of their inertness towards the N-halogenated amino acid and the aliphatic acid.

Suitable tonicity-adjusting agents include, but are not limited to, mannitol, sodium chloride, glycerin, sorbitol and the like. Suitable buffering agents include, but are not limited to, phosphates, borates, acetates and the like. Suitable surfactants include, but are not limited to, ionic and nonionic surfactants, though nonionic surfactants are preferred, RLM 100, POE 20 cetylstearyl ethers such as Procol^{®} CS20 and poloxamers such as Pluronic^{®} F68. Suitable antioxidants include, but are not limited to, sulfites, ascorbates, butylated hydroxyanisole (BHA) and butylated hydroxytoluene (BHT).

The formulations set forth herein may comprise one or more preservatives. Examples of such preservatives include p-hydroxybenzoic acid ester, alkyl-mercury salts of thiosalicylic acid, such as thiomersal, phenylmercuric nitrate, phenylmercuric acetate, phenylmercuric borate, sodium perborate, sodium chlorite, parabens such as methylparaben or propylparaben, alcohols such as chlorobutanol, benzyl alcohol or phenyl ethanol, guanidine derivatives such as polyhexamethylene biguanide, sodium perborate, or sorbic acid. In certain embodiments, the formulation may be self-preserved that no preservation agent is required.

For use in sinus and respiratory infection applications, formulations may be used that are suitable for aerosol formation using nebulizers or other such devices well known to those of skill in the art.

Some formulations are ophthalmically suitable for application to a subject's eyes. For ophthalmic administration, the formulation may be a solution, a suspension, a gel, or an ointment. In preferred aspects, formulations that include the N-halogenated amino acid and the aliphatic acid will be formulated for topical application to the eye in aqueous solution in the form of drops. The term "aqueous" typically denotes an aqueous formulation wherein the excipient is >50%, more preferably >75% and in particular >90% by weight water. These drops may be delivered from a single dose ampoule which may preferably be sterile and thus render bacteriostatic components of the formulation unnecessary. Alternatively, the drops may be delivered from a multi-dose bottle which may preferably comprise a device which extracts any preservative from the formulation as it is delivered, such devices being known in the art.

In other aspects, components may be delivered to the eye as a concentrated gel or a similar vehicle, or as dissolvable inserts that are placed beneath the eyelids. In yet other aspects, the components may be delivered to the eye as ointment, water-in-oil and oil-in-water emulsions.

For topical formulations to the eye, the formulations are preferably isotonic, or slightly hypotonic in order to combat any hypertonicity of tears caused by evaporation and/or disease. This may require a tonicity agent to bring the osmolality of the formulation to a level at or near 210-320 milliosmoles per kilogram (mOsm/kg). The pH of the solution may be in an ophthalmic acceptable range of 3.0 to 8.0. The formulations generally have an osmolality in the range of 220-320 mOsm/kg, and preferably have an osmolality in the range of 235-300 mOsm/kg. The ophthalmic formulations will generally be formulated as sterile aqueous solutions.

In certain embodiments, the N-halogenated amino acid and the aliphatic acid are formulated in a formulation that comprises one or more tear substitutes. A variety of tear substitutes are known in the art and include, but are not limited to: monomeric polyols, such as, glycerol, propylene glycol, and ethylene glycol; polymeric polyols such as polyethylene glycol; cellulose esters such hydroxypropylmethyl cellulose, carboxy methylcellulose sodium and hydroxy propylcellulose; dextrans such as dextran 70; vinyl polymers, such as polyvinyl alcohol; and carbomers, such as carbomer 934P, carbomer 941, carbomer 940 and carbomer 974P. Certain formulations of the present invention may be used with contact lenses or other ophthalmic products.

In some embodiments, the formulations set forth herein have a viscosity of 0.5-100 cps, preferably 0.5-50 cps, and most preferably 1-20 cps. This relatively low viscosity insures that the product is comfortable, does not cause blurring, and is easily processed during manufacturing, transfer and filling operations.

The N-halogenated amino acids and aliphatic acids described herein may be included in various types of formulations having activities in addition to antimicrobial activity. Examples of such formulations include: ophthalmic pharmaceutical formulations (such as ocular lubricating products and artificial tears), astringents, topical disinfectants (alone or in combination with other antimicrobial agents such as, for example, betadine, etc.) and so on.

To effectively treat various microbial infections and to minimize side-effects, the antimicrobial activity of a formulation should be maximized so that a minimum amount of active ingredient is used. The activity of the antimicrobial formulations is the result of the antimicrobial agent itself; the formulation components other than the N-halogenated amino acid normally cause little effect. The amount of the aliphatic acid required to enhance the antimicrobial activity of the N-halogenated amino acid in particular formulations can be determined by persons skilled in the art. The concentration required to enhance the antimicrobial activity of formulations while retaining acceptable safety and toxicity properties is referred to herein as "an effective amount". In certain embodiments an effective amount of aliphatic acid is about 5 mM or 0.07%. However, for safety and toxicological reasons, an effective amount can be altered higher or lower than this concentration and may be preferably in the range of about 0.0001% to 10%.

It is also contemplated that the concentrations of the ingredients comprising the formulations can vary. In preferred embodiments, the N-halogenated amino acid is present in ophthalmic formulations at a concentration of about 0.1% to 0.25% w/v. A person of ordinary skill in the art would understand that the concentrations can vary depending on the addition, substitution, and/or subtraction of ingredients in a given formulation.

Preferred formulations are prepared using an aliphatic acid buffering system that maintains the formulation at a pH of about 3 to a pH of about 8.0. In certain embodiments, topical formulations (particularly topical ophthalmic formulations, as noted above) are preferred which have a physiological pH matching the tissue to which the formulation will be applied or dispensed.

In certain embodiments, a formulation can be administered in a two-part system. For instance, the N-halogenated amino acid can be present in one part of the formulation and one or more components of the formulation are separated in a separate container or different portion of the same container until a user is ready to administer the formulation. At the instant of administration or before, the two parts may be mixed by a user. The two-part system may be useful in cases where one or more components of the formulation have stability problems when combined. Also, a two-part system may be utilized as part of a nasal/sinus spray dispensing system in certain embodiments.

### C. Route of Administration

In the methods set forth herein, administration to a subject of a pharmaceutically effective amount of a formulation that includes an N-halogenated amino acid and an aliphatic acid may be by any method known to those of ordinary skill in the art.

For example, the formulation may be administered locally, topically, intradermally, intralesionally, intranasally, subcutaneously, orally, by inhalation, by injection, by localized perfusion bathing target cells directly, via a catheter, or via lavage.

The formulation may be administered topically to an ocular surface. Regarding ophthalmic administration, it is contemplated that all local routes to the eye may be used, including topical, subconjunctival, periocular, retrobulbar, subtenon, intraocular, subretinal, posterior juxtascleral, and suprachoroidal administration.

Various otic administration techniques are also contemplated. The formulation may be delivered directly to the ear canal (for example: topical otic drops or ointments; slow release devices in the ear or implanted adjacent to the ear). Local administration routes include otic intramuscular, intratympanic cavity and intracochlear injection routes for the formulations. It is further contemplated that certain formulations may be formulated in intraotic inserts or implant devices. For instance, delivery of the formulations can be accomplished by endoscopic assisted (including laser-assisted endoscopy to make the incision into the tympanic membrane) injection into the tympanic cavity as set forth, for example, in Tsue et al., Amer. J. Otolaryngology, Vol. 16(3):158-164, 1995; Silverstein et al., Ear Nose Throat, Vol. 76:674-678, 1997; Silverstein et al., Otolaryngol Head Neck Surg, Vol. 120:649-655, 1999. Local administration can also be achieved by injection through the tympanic membrane using a fine (EMG recording) needle, through use of an indwelling catheter placed through a myringotomy incision, and injection or infusion through the Eustachian tube by means of a small tubal catheter. Furthermore, the formulations can be administered to the inner ear by placement of gelfoam or similar absorbent and adherent product soaked with the formulations against the window membrane of the middle/inner ear or adjacent structure with due discretion and caution by a skilled clinician.

Administration of the formulations described herein for the treatment of sinus tissue infection, nasal infection, upper respiratory infection, lung/lower respiratory infection, esophageal infection, and the various combinations can be via a number of methods known to those of skill in the art. Preferred administration for lower respiratory infections will be via aerosol formation by use of a nebulizer or other similar device. Formulations for the treatment of sinus infections can be administered in droplet form (often otic formulations can be used for the treatment of sinus infections) or by aerosol formation. Esophageal infections may be treated by administration of a liquid or aerosol formulation.

Other modes of administration of the formulations are via skin patches, intrapulmonary, intranasally, via liposomes formulated in an optimal manner, and via slow release depot formulations. Various devices can be used to deliver the formulations to the affected ear compartment; for example, via catheter or as exemplified in U.S. Patent No. 5,476,446 which provides a multi-functional apparatus specifically designed for use in treating and/or diagnosing the inner ear of the human subject. Also see U.S. Patent No. 6,653,279 for other devices for this purpose.

### V. Examples

The following examples are presented to further illustrate selected embodiments of the present invention. Examples 1-2 below were prepared according to embodiments of the present invention.

### EXAMPLE 1

| **Ingredient** | **% w/v** |
|---|---|
| Sodium 2,2-dimethyl-N,N-dichlorotaurine | 0.1 |
| Sodium Acetate Trihydrate | 0.07 |
| Sodium Chloride | 0.8 |
| Hydrochloric Acid | q.s. pH 4 |
| Sodium Hydroxide | q.s. pH 4 |
| Purified Water | q.s. 100% |

### EXAMPLE 2

| **Ingredient** | **% w/v** |
|---|---|
| Sodium 2,2-dimethyl-N,N-dichlorotaurine | 0.1 |
| Sodium Acetate Trihydrate | 0.07 |
| Sodium Chloride | 0.8 |
| Hydrochloric Acid | q.s. pH 5.5 |
| Sodium Hydroxide | q.s. pH 5.5 |
| Purified Water | q.s. 100% |

### EXAMPLE 3

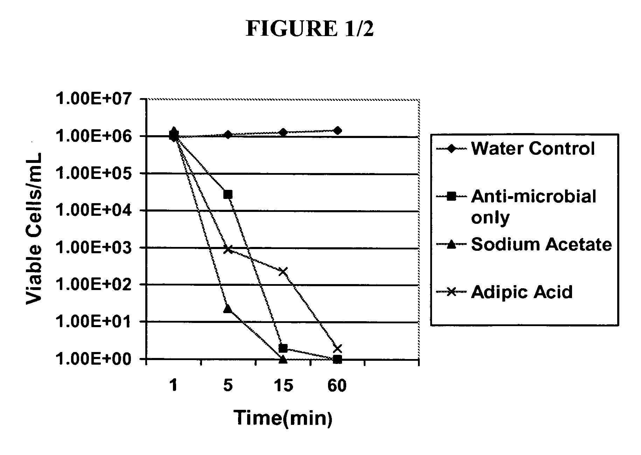

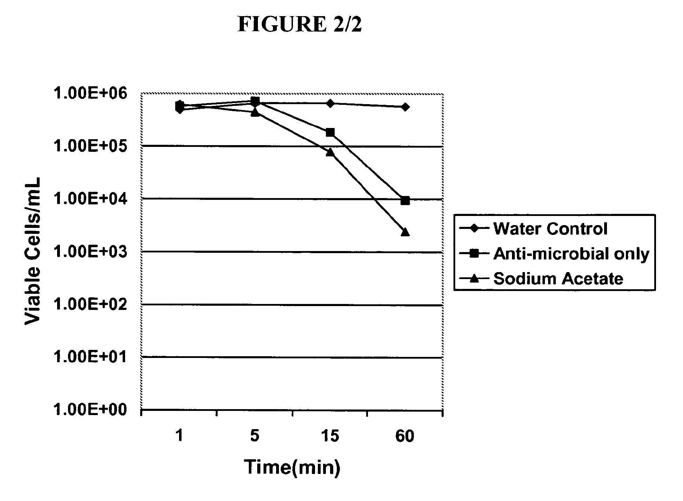

The antimicrobial activity of the formulations according to embodiments of the present invention were evaluated by a standard microbiological analysis. The results of this evaluation are summarized in Tables 1 and 2 below and are shown graphically in FIGURES 1 and 2. For the evaluation, bacterial and fungal isolates were grown overnight on appropriate agar media as source of fresh cells. A suspension of these fresh cells was prepared in saline at approximately 1 X 10⁸ cfu/mL. These suspensions were added directly to the test agents (various solutions of sodium 2,2-dimethyl-N,N-dichlorotaurine and control solutions). The initial concentration of cells in the test agent solutions was approximately 1 X 10⁶ cfu/mL. The exposure of microorganisms to the test agent was conducted at room temperature for up to 60 minutes. At selected times, an aliquot was withdrawn and diluted into phosphate buffered saline at 4°C. Viability was determined following serial dilution and filtration onto Milliflex cassettes.

**TABLE 1 - Evaluation with S. aureus**

| **Product** | **Sampling Time (min)** | **# Colonies** | **Correction Factor** | **Dilution Factor** | **Viable Cells/ml** | **% Survivors** |
|---|---|---|---|---|---|---|
| | 0 | 103 | 1.11 | 10000 | 1143300 | 100.00 |
| Control (water) | 5 | 91 | 1.23 | 10000 | 1119300 | 97.90 |
| | 15 | 108 | 1.23 | 10000 | 1328400 | 116.19 |
| | 60 | 102 | 1.23 | 10000 | 1254600 | 109.73 |
| | 0 | 128 | 1.11 | 10000 | 1420800 | 100.00 |
| Vehicle | | | | | | |
| pH 4.0 w/sodium acetate | 60 | 79 | 1.23 | 10000 | 971700 | 68.3910 |
| | 180 | 88 | 1.23 | 10000 | 1082400 | 76.1824 |
| | 1440 | 101 | 1.23 | 10000 | 1242300 | 87.4367 |
| | 0 | 97 | 1.11 | 10000 | 1076700 | 100.00 |
| 2,2-dimethyl-N,N-dichlorotaurine 0.001% | | | | | | |
| pH 4.0 No Buffer | 5 | 223 | 1.23 | 100 | 27429 | 2.55 |
| | 15 | 2 | 1.11 | 1 | 2.22 | 0.0002 |
| | 60 | 0 | 1.11 | 1 | 0 | 0.00 |
| | 0 | 128 | 1.11 | 10000 | 1420800 | 100.00 |
| 2,2-dimethyl-N,N-dichlorotaurine 0.001 % | | | | | | |
| pH 4.0 w/sodium acetate | 5 | 21 | 1.11 | 1 | 23.31 | 0.001641 |
| | 15 | 1 | 1.11 | 1 | 1.11 | 0.000078 |
| | 60 | 0 | 1.11 | 1 | 0 | 0.000000 |
| | 0 | 98 | 1.11 | 10000 | 1087800 | 100.00 |
| 2,2-dimethyl-N,N-dichlorotaurine 0.001% | | | | | | |
| pH 4.0 w/adipic acid | 5 | 74 | 1.23 | 10 | 910.2 | 0.084 |
| | 15 | 210 | 1.11 | 1 | 233.1 | 0.021 |
| | 60 | 2 | 1.11 | 1 | 2.22 | 0.0002 |
| | 0 | 92 | 1.11 | 10000 | 1021200 | 100.00 |
| Vehicle | | | | | | |
| pH 5.5 w/sodium acetate | 5 | 90 | 1.23 | 10000 | 1107000 | 108.402 |
| | 15 | 87 | 1.23 | 10000 | 1070100 | 104.788 |
| | 60 | 102 | 1.23 | 10000 | 1254600 | 122.855 |
| | 0 | 92 | 1.11 | 10000 | 1021200 | 100.00 |
| 2,2-dimethyl-N,N-dichlorotaurine 0.001% | | | | | | |
| pH 5.5 w/sodium acetate | 5 | 104 | 1.23 | 1000 | 127920 | 12.5264 |
| | 15 | 82 | 1.23 | 100 | 10086 | 0.987662 |
| | 60 | 222 | 1.23 | 10 | 2730.6 | 0.267391 |
| | 0 | 114 | 1.11 | 10000 | 1265400 | 100.00 |
| 2,2-dimethyl-N,N-dichlorotaurine 0.001% | | | | | | |
| pH 5.5 w/adipic acid | 5 | 82 | 1.23 | 10000 | 1008600 | 79.71 |
| | 15 | 118 | 1.23 | 1000 | 145140 | 11.47 |
| | 60 | 81 | 1.23 | 100 | 9963 | 0.79 |

The anti-infective activity of the N-halogenated amino acid 2,2-dimethyl-N,N-dichlorotaurine, as measured by the number of viable cells per mL of *S. aureus,* was dramatically improved when the formulation contained sodium acetate. As shown above in Table 1, there were only 24 viable cells per mL measured 5 minutes after treatment with 0.001% 2,2-dimethyl-N,N-dichlorotaurine formulated with acetate buffer at pH 4. In contrast, there were 27429 and 910 viable cells per mL 5 minutes after treatment with a 0.001% 2,2-dimethyl-N,N-dichlorotaurine formulation comprising no buffer and adipic acid buffer, respectively. The results indicate that the acetate compound formulations increase the antimicrobial activity by nearly 2 log steps relative to adipic acid buffer, and by more than 3 log steps relative to a no buffer formulation.

FIGURE 1 graphically illustrates the results shown above in Table 1. The graph clearly shows that the antimicrobial activity of an N-halogenated amino acid, 2,2-dimethyl-N,N-dichlorotaurine was increased when formulated with a sodium acetate buffer.

**TABLE 2 -Evaluation with C. albicans**

| **Product** | **Sampling Time (min)** | **# Colonies** | **Correction Factor** | **Dilution Factor** | **Viable Cells/ml** | **% Survivors** |
|---|---|---|---|---|---|---|
| | 0 | 44 | 1.11 | 10000 | 488400 | 100.00 |
| Control (water) | 5 | 59 | 1.11 | 10000 | 654900 | 134.09 |
| | 15 | 59 | 1.11 | 10000 | 654900 | 134.09 |
| | 60 | 51 | 1.11 | 10000 | 566100 | 115.91 |
| | 0 | 52 | 1.11 | 10000 | 577200 | 100.000 |
| Vehicle | | | | | | |
| pH 4.0 w/sodium acetate | 5 | 58 | 1.11 | 10000 | 643800 | 111.538 |
| | 15 | 58 | 1.11 | 10000 | 643800 | 111.538 |
| | 60 | 59 | 1.11 | 10000 | 654900 | 113.462 |
| | 0 | 52 | 1.11 | 10000 | 577200 | 100.000 |
| 2,2-dimethyl-N,N-dichlorotauri ne 0.001% | | | | | | |
| pH 4.0 No Buffer | 5 | 65 | 1.11 | 10000 | 721500 | 125.000 |
| | 15 | 166 | 1.11 | 1000 | 184260 | 31.923 |
| | 60 | 86 | 1.11 | 100 | 9546 | 1.654 |
| | 0 | 56 | 1.11 | 10000 | 621600 | 100.0000 |
| 2,2-dimethyl-N,N-dichlorotauri ne 0.001% | | | | | | |
| pH 4.0 w/sodium acetate | 5 | 40 | 1.11 | 10000 | 444000 | 71.4286 |
| | 15 | 71 | 1.11 | 1000 | 78810 | 12.6786 |
| | 60 | 22 | 1.11 | 100 | 2442 | 0.3929 |

Similar results are seen in evaluations of acetate compound formulations against *C. albicans.*

The anti-infective activity of the N-halogenated amino acid 2,2-dimethyl-N,N-dichlorotaurine, as measured by the number of viable cells per mL of *C. albicans,* was dramatically improved when the formulation contained sodium acetate. As shown above in Table 1, there were only 2442 viable cells per mL measured 60 minutes after treatment with 0.001% 2,2-dimethyl-N,N-dichlorotaurine formulated with acetate buffer at pH 4. In contrast, there were 9546 viable cells per mL 60 minutes after treatment with a 0.001 % 2,2-dimethyl-N,N-dichlorotaurine formulation comprising no buffer, respectively. The results indicate that the acetate compound formulations have less than one third of the viable cells per mL of the formulations without acetate.

FIGURE 2 graphically illustrates the results shown above in Table 2. The graph clearly shows that the antimicrobial activity of an N-halogenated amino acid, 2,2-dimethyl-N,N-dichlorotaurine was increased when formulated with a sodium acetate buffer.

## Claims

1. Non-therapeutic use of an aliphatic acid for improving the antimicrobial activity of a formulation comprising a N-halogenated amino acid.

2. An aliphatic acid for use in a method of improving the antimicrobial activity of a formulation comprising a N-halogenated amino acid for treating mammalian and human subjects having or at risk of having a microbial tissue infection.

3. An aliphatic acid for use according to claim 2 or the non-therapeutic use of claim 1 wherein the aliphatic acid is selected from the group consisting of:
sodium acetate, potassium acetate, calcium acetate, magnesium acetate, butyric acid and combinations thereof.

4. An aliphatic acid for use according to claim 2 or the non-therapeutic use of claim 1 wherein the N-halogenated amino acid is a chlorotaurine.

5. An aliphatic acid for use according to claim 2 or the non-therapeutic use of claim 1 wherein the chlorotaurine is sodium 2,2-dimethyl-N,N-dichlorotaurine.

6. An aliphatic acid for use according to claim 2 or the non-therapeutic use of claim 1 wherein the N-halogenated amino acid forms an ion-pair with a component of said formulation.

7. A method for disinfecting and/or cleaning a contact lens comprising:
contacting a contact lens with a formulation comprising a N-halogenated amino acid and an aliphatic acid for a time sufficient to disinfect and/or clean the lens.

## Patentansprüche

1. Nicht-therapeutische Verwendung einer aliphatischen Säure zur Verbesserung der antimikrobiellen Aktivität einer Formulierung, umfassend eine N-halogenierte Aminosäure.

2. Aliphatische Säure zur Verwendung in einem Verfahren zur Verbesserung der antimikrobiellen Aktivität einer Formulierung, umfassend eine N-halogenierte Aminosäure, zur Behandlung von zu den Säugetieren gehörigen und menschlichen Probanden, die eine durch Mikroorganismen verursachte Gewebeinfektion aufweisen oder bei denen ein Risiko für eine solche besteht.

3. Aliphatische Säure zur Verwendung nach Anspruch 2 oder zur nicht-therapeutischen Verwendung nach Anspruch 1, wobei die aliphatische Säure aus der Gruppe ausgewählt ist, bestehend aus:
Natriumacetat, Kaliumacetat, Calciumacetat, Magnesiumacetat, Buttersäure und Kombinationen davon.

4. Aliphatische Säure zur Verwendung nach Anspruch 2 oder zur nicht-therapeutischen Verwendung nach Anspruch 1, wobei die N-halogenierte Aminosäure ein Chlortaurin ist.

5. Aliphatische Säure zur Verwendung nach Anspruch 2 oder zur nicht-therapeutischen Verwendung nach Anspruch 1, wobei das Chlortaurin Natrium-2,2-dimethyl-N,N-dichlortaurin ist.

6. Aliphatische Säure zur Verwendung nach Anspruch 2 oder zur nicht-therapeutischen Verwendung nach Anspruch 1, wobei die N-halogenierte Aminosäure ein Ionenpaar mit einer Komponente der Formulierung bildet.

7. Verfahren zum Desinfizieren und/oder Reinigen einer Kontaktlinse, umfassend:
das Kontaktieren einer Kontaktlinse mit einer Formulierung, umfassend eine N-halogenierte Aminosäure und eine aliphatische Säure, für eine Zeit, die ausreicht, um die Linse zu desinfizieren und/oder zu reinigen.

## Revendications

1. Utilisation non thérapeutique d'un acide aliphatique pour améliorer l'activité antimicrobienne d'une formulation comprenant un acide aminé N-halogéné.

2. Acide aliphatique à utiliser dans un procédé d'amélioration de l'activité antimicrobienne d'une formulation comprenant un acide aminé N-halogéné pour traiter des sujets mammifères et humains présentant ou risquant de présenter une infection tissulaire microbienne.

3. Acide aliphatique à utiliser selon la revendication 2 ou utilisation non thérapeutique selon la revendication 1, dans lequel ou laquelle l'acide aliphatique est choisi dans le groupe constitué de :
l'acétate de sodium, l'acétate de potassium, l'acétate de calcium, l'acétate de magnésium, l'acide butyrique et leurs combinaisons.

4. Acide aliphatique à utiliser selon la revendication 2 ou utilisation non thérapeutique selon la revendication 1, dans lequel ou laquelle l'acide aminé N-halogéné est une chlorotaurine.

5. Acide aliphatique à utiliser selon la revendication 2 ou utilisation non thérapeutique selon la revendication 1, dans lequel ou laquelle la chlorotaurine est la sodium 2,2-diméthyl-N,N-dichlorotaurine.

6. Acide aliphatique à utiliser selon la revendication 2 ou
utilisation non thérapeutique selon la revendication 1, dans lequel ou laquelle l'acide aminé N-halogéné forme une paire d'ions avec un composant de ladite formulation.

7. Procédé pour désinfecter et/ou nettoyer une lentille de contact, comprenant :
mettre une lentille de contact en contact avec une formulation comprenant un acide aminé N-halogéné et un acide aliphatique pendant une durée suffisante pour désinfecter et/ou nettoyer la lentille.
